# EUROPEAN PATENT APPLICATION

(11) **EP 3 726 834 A1**
(43) Date of publication of application: **21.10.2020**
(21) Application number: 19169896.8
(22) Date of filing: 17.04.2019
(51) Int. Cl.: H04N 7/14, G16H 30/20, G16H 80/00

(54) **TELEPRESENCE SYSTEM AND METHOD**

(71) Applicant: Medneo GmbH, 10117 Berlin (DE)
(72) Inventor: Shahal, Avner, 12107 Berlin (DE); Juneja, Medha, 10781 Berlin (DE); Dr. Hartkens, Thomas, 13086 Berlin (DE); Vogt, Johannes, 10827 Berlin (DE); Martins, Ronald, 12207 Berlin (DE); Dr. Issing, Matthias, 10627 Berlin (DE); Dr. Liebel, Urban, 69234 Dielheim (DE)
(74) Representative: Laqua, Bernd Christian Kurt

(57) **Abstract**

A telepresence system (10) and a telepresence method for teleradiology is disclosed. The telepresence system comprises an acquisition system located at a radiological diagnostic site and comprising one or more acquisition devices (14, 16, 18), wherein each acquisition device (14, 16, 18) is configured to acquire a respective video stream (20, 22, 24) comprising video data acquired of a region (26, 28, 30) of the diagnostic site at which the respective acquisition device (14, 16, 18) is located, and wherein the acquisition system is configured to transmit the one or more video streams (20, 22, 24) to a controller (12), a user interface system (50) located remotely from the diagnostic site, wherein the user interface system (50) comprises a user communication input unit (52) and a replay unit with an immersive display (54), and a user communication output system (62, 64, 66) located at the diagnostic site. The controller (12) is configured to receive the one or more video streams (20, 22, 24) and to transmit at least one of the video streams to the user interface system (50), wherein the replay unit is configured to output the at least one video stream on the immersive display (54) to a user (70), and the user communication input unit (52) is configured to receive a user input and generate a user input signal representative of the user input, wherein the user interface system (50) is configured to transmit the user input signal to the controller (12), and the controller (12) is configured to receive the user input signal and to transmit the user input signal to the user communication output system (62, 64, 66) for output of the user input at the diagnostic site.

## Description

### Technical Field

The present disclosure generally relates to a telepresence system and a telepresence method for teleradiology. More specifically, the present disclosure concerns an immersive telepresence system and method.

### Background

Telepresence is the ability to interact with an environment which is physically remote from the actual location of the user. Albeit such interaction can be performed at several levels of complexity, in the context of the present disclosure, telepresence may be understood as enabling the user to at least visually perceive a part of the environment remote from the user's location. Telepresence implementations in the field of radiology are referred to by the term "teleradiology", which is generally defined as radiology involving the transmission of radiologic image data to a remote location.

Radiological examinations are normally performed according to so-called standard operating procedures (SOP). They comprise detailed instructions for each step of the examination, including preparatory steps, and aim at achieving a standardized high radiological imaging performance. Exemplary instructions comprised in such standard operating procedure relate to how the subject (e.g., proband or patient) is to be positioned and which imaging parameters are to be selected for which medical indication.

After medical staff prepares and appropriately positions the subject for the actual radiological imaging (i.e., the image acquisition) in a preparation area of a radiological diagnostic site (e.g., a radiological department of a hospital), the subject is transferred to the so-called imaging modality room in which the medical imaging device (e.g., an X-ray CT or MRT scanner) is located. The examination is then performed by a medical, in particular radiological, technician who selects predefined imaging/scanning sequences according to the imaging modality type, body region and examination objective in line with the standard operating procedures. Upon completion of the examination, the images are normally stored for the physician to enable her/him to make the diagnosis and report thereon, and the subject is finally discharged from the medical imaging device.

In teleradiology, the physician is not present at the radiological diagnostic site, but can make the diagnosis from a remote location. For example, a radiologist staying at home during stand-by duty, i.e., located remotely from a radiological department of a hospital, can access the computer system of the radiological department to display and interact with a duplicated screen of a medical technician's or another physician's desktop (desktop sharing). The radiologist can thus inspect the information shown on the medical technician's or other physician's desktop, and interpret the information. In addition, the radiologist can access from home a server of a Picture Archiving and Communication System (PACS) in which radiological images of a subject (e.g., patient) are stored, to obtain further details on the subject. Via telephone, the radiologist can then provide the medical technician or physician with feedback or support, in particular, with a diagnosis based on the interpretation. Further, if the subject of interest is not appropriately positioned in the employed imaging device, the remote radiologist can provide the medical technician with instructions for how to better position the subject.

Against this background, it is an object of the present disclosure to provide a telepresence system and a telepresence method which allow for more intuitively and efficiently performing a radiological examination.

### Summary

This object is achieved by a telepresence system and a telepresence method according to the independent claims. The telepresence system is provided for teleradiology and comprises a controller, an acquisition system, a user interface system, and a user communication output system. The acquisition system and the user communication output system are located at a radiological diagnostic site, and the user interface system is located remotely from the diagnostic site. The acquisition system comprises one or more acquisition devices, wherein each acquisition device is configured to acquire a respective video stream with video data acquired of a region of the diagnostic site at which the respective acquisition device is located. The acquisition system is configured to transmit the one or more video streams to the controller. The user interface system comprises a user communication input unit and a replay unit with an immersive display.

The controller is configured to receive the one or more video streams and to transmit at least one of the video streams to the user interface system, and the replay unit is configured to output the at least one video stream on the immersive display to a user. The user communication input unit is configured to receive a user input and generate a user input signal representative of the user input, the user interface system is configured to transmit the user input signal to the controller, and the controller is configured to receive the user input signal and to transmit the user input signal to the user communication output system for output of the user input at the diagnostic site. The at least one video stream may, in particular, be a plurality of video streams. These video streams may be simultaneously displayed on the immersive display.

The telepresence system may not only provide the user (i.e., the remote physician/radiologist) with a more complete insight on the procedure ongoing at the radiological diagnostic site, but may also enable her/him to more efficiently contribute to improving the radiological examination. The user may not have to wait until the medical technician or other physician at the radiological diagnostic site has finished the examination until the user can see whether the patient has been appropriately prepared and positioned for the scan. Rather, the user can follow the procedure on the immersive display and can, if necessary, directly instruct the medical technician at the radiological diagnostic site how to correct any inadequacies in the preparation, positioning of the subject, and the imaging procedure. Specifically, the remote user can check whether the SOP is correctly followed, so that the risk of acquiring unsuitable images or even harming the subject can be effectively reduced. Hence, the user can more fully participate in the entire medical examination. Even further, the remote user can immerse herself/himself into areas such as an X-ray CT imaging room in which the subject/patient is currently scanned, i.e., into areas not to be accessed even by local staff during the image acquisition.

The user input may comprise a textual input, an audio input and/or a video input. The user communication output system may specifically be arranged at the region of the diagnostic site at which the subject or on-site medical staff can be located. Thus, the user input (text/sound/video) may be conveyed to the region of the diagnostic site at which the subject and/or on-site medical staff is located and output by means of the user communication output system. Thereby, the burden on the user for communicating with the subject or on-site medical staff may significantly be reduced. Furthermore, the one or more video streams may, in addition to the video data, comprise audio data acquired of the region of the diagnostic site at which the respective acquisition device is located. This may enable the user to more thoroughly immerse herself/himself into the region of the diagnostic site.

Each one of the acquisition devices may be a video camera. Preferably, one or more of the acquisition devices, in particular all acquisition devices, is/are configured to acquire the respective video stream as a real-time video stream and to transmit it via the controller to the replay unit in real-time. In this case, it may be-preferred that the replay unit is configured to output the at least one video stream in real-time, too. In other words, the video stream transmitted to the user and output on the immersive display may be a live video stream of the region of the diagnostic site at which the respective acquisition device is located. If the video stream comprises audio and video data, the view and sound of the region of the radiological diagnostic site at which the respective acquisition device is located may both be transmitted in the live video stream.

Vice versa, the generation of the user input signal representative of the user input may preferably be performed in real-time, too. In this case, the user interface system may transmit the user input signal to the controller in real-time, and the controller may be configured to receive the user input signal and transmit it to the user communication output system in real-time so that the user communication output system outputs the user input at the diagnostic site in real-time. Thus, especially when the user input comprises an audio and/or video input which is transferred to and output by the user communication output system, the user can precisely instruct and assist the persons at the region of the diagnostic site without delays, so that the duration of the radiological examination and, thus, the burden on the patient may be reduced. Advantageously, the controller may further be configured to transmit the at least one video stream to the user interface system simultaneously with transmitting the user input signal to the user communication output system to expedite the examination.

In the present context, an immersive display may be a display which enables the user to feel (visually or audio-visually) immersed in the respective region of the diagnostic site, when she/he is located remotely from the diagnostic site and in a designated spatial relationship with the display. Insofar, the immersive display may be adapted to make the user believe to be present at the region of the diagnostic site, i.e., perceive the environment at the region of the diagnostic site as the user's real environment, and disregard being at the remote location.

In one variant, the immersive display may be (or comprise) virtual reality goggles. The virtual reality goggles may comprise positional and rotational sensors configured to detect a movement of the goggles. In particular, when the virtual reality goggles are arranged in a first position, they may be configured to display a first part of the video data, wherein the first part may be acquired of a first section of the region of the diagnostic site. When the virtual reality goggles are arranged in a second position different from the first position, they may be configured to display a second part of the video data, wherein the second part may be acquired of a second section of the region of the diagnostic site. Preferably, the second section may be different from, in particular, may not overlap with the first section.

It will be understood that other implementations of the immersive display are generally conceivable. For example, the immersive display may comprise a curved display panel extending azimuthally along an arc (angular range) of more than a quarter circle (90°), preferably, along an arc of more than one third (120°) of a circle, or along an arc of more than a half circle (180°). When the user is situated at the center of the circle (i.e., at the designated viewing position), thus, in a designated spatial relationship with the display, the immersive display azimuthally may extend over the majority of (i.e., more than half of) the natural/physical visual field of the user (about 180°), so that the user feels immersed in the region of the diagnostic site. Moreover, the curved display panel may extend in elevation over more than half of the elevational natural/physical visual field of the user (about 130°), i.e., over more than 65°, more than 70°, more than 85° or more than 90°. Accordingly, the curved display panel may be curved horizontally and/or vertically.

The immersive display may further comprise a visual field restricting device configured to restrict the visual field to a visual field narrower than the natural/physical visual field of the user. In this case, the curved display panel may preferably extend azimuthally or in elevation beyond the restricted visual field when the user is situated at the designated viewing position and looks towards the center of the display panel.

At least one of the acquisition devices may be configured for acquiring video data of at least a half sphere, preferably a full sphere, around the acquisition device, and/or stereoscopic image data so that the video data may be stereoscopic video data. In particular, it may be preferred that the video data is acquired of a field of view which corresponds to the dimension of the immersive display in case of outputting the video stream on the curved display panel. In other words, if the immersive display comprises the curved display panel extending azimuthally over an angular range specified above, the azimuthal field of view may advantageously extend over the same angular range (e.g., more than 90°, 120° or 180°).

The radiological diagnostic site may be a radiology department in a hospital or a radiological practice, and may comprise a plurality of different areas such as different rooms. Examples of such areas are a consulting room, a preparation area adapted for preparing a subject for radiological examination, an examination room adapted for controlling the examination, and an imaging modality room adapted for radiologically imaging the subject. In the consulting room, the subject may be briefed for the examination and, in the preparation room, the subject may be appropriately positioned on a patient bed and prepared for the examination, for example by applying an intravenous access for injection of a contrast agent. The examination room may comprise one or more displays associated with a Radiological Information System (RIS), a Hospital Information System (HIS), a Picture Archiving and Communication System (PACS) and/or control display/s for the imaging apparatus arranged in the imaging modality room (i.e., the actual medical imaging room).

The acquisition devices may preferably be arranged at the diagnostic site so as to acquire the video data, and optionally the audio data, of different areas of the diagnostic site. Optionally, user communication output devices of the user communication output system are located in the different areas for outputting the user input in these areas. Thus, the remote user's input can be conveyed to on-site staff irrespective of the area they are located in. Advantageously, the user can also communicate with the subject, for example, in order to calm her/him down before the examination. Further, each video stream may be acquired by another one of the acquisition devices. For example, a first acquisition device may be arranged in a preparation room for acquiring video data of a first region in which the patient bed is located during the preparation. A second acquisition device may be arranged in the examination room for acquiring video data of a second region in which the control displays and/or displays associated with the PACS/RIS/HIS are located. A third acquisition device may be arranged in the imaging modality room for acquiring video data of a third region in which the imaging apparatus is located.

Further, the user interface system may be configured to receive a first user command for selecting the at least one video stream from among the video streams, and to transmit, to the controller, a video selection signal based on the first user command. In this case, the controller may be configured to select the at least one video stream based on the video selection signal. Accordingly, the user can swiftly, such as in real-time, switch between the different regions and/or areas. Nevertheless, the user can immerse herself/himself into the region/area of interest. This circumstance enables the user to supervise the radiological examination efficiently. Further, if the radiological diagnostic site has capacities for simultaneously examining a plurality of subjects, the user can switch between the locations at which these subjects are located.

In a further variant, the system may comprise one or more radiological systems located at the radiological diagnostic site. Examples of such radiological systems may comprise a medical imaging device, a Radiological Information System, RIS, the Picture Archiving and Communication System, PACS, a Hospital Information System, HIS, a patient positioning assistance system, and an intravascular injector, such as a contrast agent injector. The medical imaging device may be an X-ray CT imaging device, an MRT imaging device, a PET-CT imaging device, a PET-MRT imaging device, or an ultrasonic imaging device. These imaging devices may comprise, in addition to a scanning unit, a control unit for controlling the scanning unit. Preferably, the scanning unit may be arranged in the imaging modality room and a user interface system of the control unit may be located in the examination room. The radiological information system may be a computing system for documentation and administration of medical and administrative data, inter alia, for organizing appointments and for resource management. The intravascular injector may be configured for administering the intravascular injection (in particular, intravenous injection) of a fluid into a blood vessel of the subject. The fluid may comprise a contrast agent determined based on the type of utilized medical imaging device so as to increase the contrast of the blood vessel over the tissue surrounding the blood vessel. For example, the contrast agent may comprise iodine if the imaging device is based on X-rays and may comprise gadolinium if the imaging device is based on magnetic resonance. For example, the intravascular injector may be a perfusor with a perfusion pump.

The one or more radiological systems may each be configured to transmit a radiological system video feed of a display screen of the respective at least one radiological system to the controller. Furthermore, the controller may be configured to overlay the radiological system video feed/s on the at least one video stream to be transmitted to the user interface system so as to augment the at least one video stream. This overlaying may be triggered by a second user command to be input by means of the user communication input unit. In particular, the user interface system may be configured to receive a second user command for selecting one of the one or more radiological systems and to transmit, to the controller, a radiological system selection signal based on the selection. In response to receiving the radiological system selection signal, the controller may overlay the selected radiological system video feed on the at least one video stream.

In more detail, the controller may be configured to start the overlay of the radiological system video feed in response to receiving the radiological system selection signal, and to stop the overlay of the radiological system video feed in response to receiving a further signal corresponding to a further user command for stopping the overlay. Optionally, the controller may further overlay an icon (so called "hot spot") on the at least one video stream, and the overlaying may be triggered when the user selects this icon by means of the user communication input unit. In addition, the controller and the user interface system may be configured such that the user may interact with the radiological systems whose video feed/s are currently displayed on the immersive display 54. It is noted that a plurality of such radiological system video feeds may be simultaneously overlaid on the video stream displayed on the immersive display 54, providing the user with a better overview of the situation.

Regardless of whether the immersive display device comprises the curved display panel or the virtual reality goggles, it is conceivable that the region of the diagnostic site comprises a display screen shown on a display device arranged in the region of the diagnostic site. If the region of the diagnostic site comprises at least one display associated with one or more of the radiological systems, it may be preferred that the radiological system video feed is overlaid on the at least one video stream so that the representation of the at least one display in the video stream is at least partially covered. Advantageously, the overlay may be provided such that the user frontally views the radiological system video feed / the representation of the display screen of the respective at least one radiological system.

The controller may be configured to overlay acquired radiological images over the at least one video stream so as to be shown to the user arranged prospectively and effectively side by side, preferably, along a circular arc, wherein the circular arc may be centered at the designated viewing position. Specifically, when the system comprises a radiological system in the form of a Picture Archiving and Communication System, PACS, the radiological system video feed may be overlayed on the at least one video stream such that radiological images are arranged side by side along the circular arc, for example. If the region of the diagnostic site comprises a display associated with the PACS, the radiological system video feed may be overlaid so as to extend beyond the representation of the display associated with the PACS on the immersive display. The radiological images may be images according to the digital imaging and communications in medicine (DICOM) standard (or simply "DICOM images"). Further, the radiological images may be displayed side by side, in particular, for comparing the images from different examinations of the same subject. As such, radiological images from different examinations of the same subject may be displayed side by side.

In a further variant, the controller may be configured to process the at least one video stream for anonymization of persons appearing in the video stream before outputting the processed at least one video stream on the immersive display. Processing for anonymization may comprise face recognition and low-pass filtering, for example. Specifically, each image of the at least one video stream may be processed by determining a first portion of the image with a face of a person as well as processing this first portion so that the face features may be invisible on the display. This latter processing may comprise low-pass filtering the first portion, or replacing the first portion with a predetermined graphical item, for example. Furthermore, it may be conceivable to replace each person in the image of the video streams by an avatar.

In addition, the user communication input unit may be designed in the same manner as the acquisition system, i.e., comprise features corresponding to the features of the acquisition system, and the user communication output system may be designed in the same manner as the immersive display, i.e., comprise features corresponding to the features of the immersive display.

The telepresence method comprises the steps of acquiring, by each of one or more acquisition devices of an acquisition system located at a radiological diagnostic site, a respective video stream, wherein each video stream comprises video data acquired of a region of the diagnostic site at which the respective acquisition device is located, transmitting, by the acquisition system, the one or more video streams to a controller, receiving, by the controller, the one or more video streams, transmitting, by the controller, at least one of the video streams to a user interface system located remote from the diagnostic site, outputting, by a replay unit of the user interface system, the at least one video stream on an immersive display of the replay unit to a user, receiving, by a user communication input unit of the user interface system, a user input, generating, by the user communication input unit, a user input signal representative of the user input, transmitting, by the user interface system, the user input signal to the controller, receiving, by the controller, the user input signal, and transmitting, by the controller, the user input signal to a user communication output system located at the diagnostic site for output at the diagnostic site.

The method may further comprise method steps corresponding to any of the above described functions of the telepresence system.

### Brief Description of the Drawings

A preferred embodiment of a telepresence system according to the present disclosure is now described in further detail by referring to the enclosed schematic drawings, wherein
- Fig. 1: shows an embodiment of a telepresence system for radiology;
- Fig. 2: shows a user point of view in the telepresence system of Fig. 1, wherein three different video streams are illustrated that can be selected for output to the user; and
- Fig. 3: shows a detailed view of the telepresence system of Fig. 1, wherein a video stream and a radiological system video feed are shown.

### Detailed Description

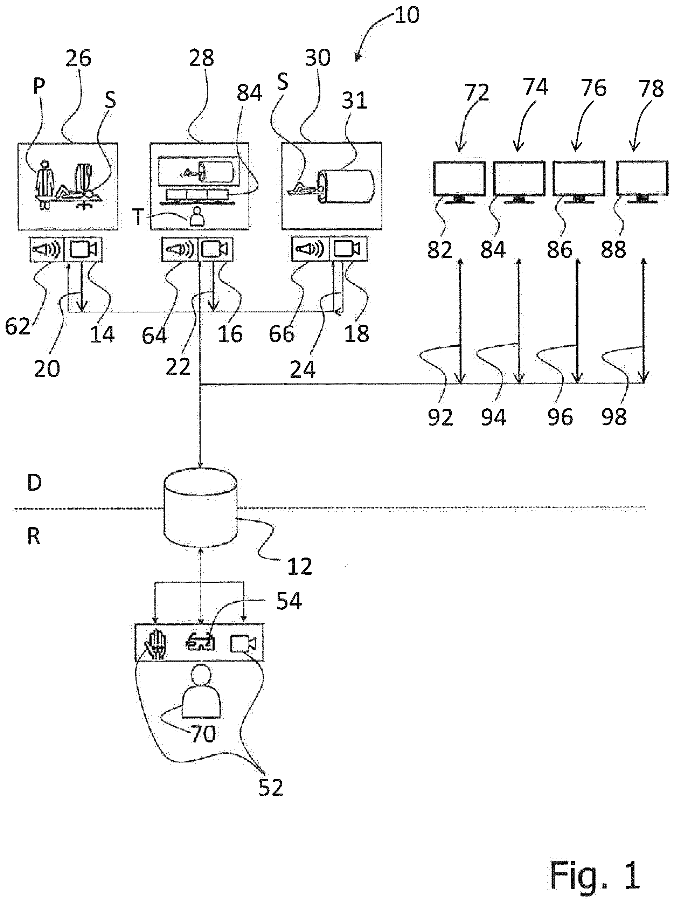

Figures 1 to 3 show a telepresence system 10 for teleradiology comprising a controller 12, an acquisition system with a first acquisition device 14, a second acquisition device 16 and a third acquisition device 18, a user interface system 50 having at least one user communication input unit 52 and a replay unit with an immersive display 54, and a user communication output system comprising user communication output units 62, 64 and 66. The acquisition system may comprise a larger or smaller number of acquisition devices, and is located at a radiological diagnostic site D, which may be a radiology department in a hospital or, in general, a radiological center. The user communication output system 62, 64, 66 is located at the diagnostic site D, too. The user interface system 50, on the other hand, is positioned at a remote location R which is located remotely from the diagnostic site D. In the drawings, the diagnostic site D has a border with the remote location R, which is randomly depicted to be at the controller 12. It will be understood, however, that the diagnostic site D and the remote location R can be separated by a larger distance from each another (e.g., the diagnostic site D and the remote location R may be in different buildings or even cities). Furthermore, if there is a common border, this border can be at locations, such as the outer walls of the building in which the region of the diagnostic site D is located, at the border of the diagnostic site D, or at the border of a radiology department of a medical center / hospital, for example.

The first acquisition device 14 is adapted to acquire a first video stream 20 with video data and audio data of a first region 26 of the diagnostic site D as real-time video stream. Herein, the first region 26 is exemplarily a preparation room in which a subject S (e.g., a patient) can be prepared for the radiological examination by positioning the subject S on a patient bed in a position appropriate for the radiological scan, optionally by providing the subject S with an intravenous access for injection of a contrast agent, for example. In particular, the first acquisition device 14 is located at the first region 26 and may be given by a 360° camera. A 360° camera is a camera which is configured to have a field of view of 4π around the camera, i.e., the 360° camera acquires the video data in an azimuthal range of 360° and an elevational range of 180° around the camera. To do so, it may comprise a plurality of lenses and image acquisition sensors such as CCD sensors, and may combine the images / videos acquired by these image acquisition sensors. Specifically, the first acquisition device 14 may be positioned inside the preparation room in the proximity of the patient bed.

The second acquisition device 16 is adapted to acquire a second video stream 22, with video and audio data of a second region 28 of the diagnostic site D as real-time video stream. In this embodiment, the second region 28 is exemplarily a radiological examination room for a radiological technician T controlling the imaging procedure in accordance with an SOP. In the second region 28, a display screen 82 of a Radiological Information System 72, RIS, a display screen 84 of a Picture Archiving and Communication System 74, PACS, and a display screen 86 of a medical imaging device 76 are arranged, for example. For illustration of these systems, the display screens are additionally shown on the right-hand side of Fig. 1. The second acquisition device 16 is herein a further 360° camera located at the second region, in particular centrally in the radiological examination room.

The third acquisition device 16 is adapted to acquire a third video stream 22, with video and audio data of a third region 30 of the diagnostic site D as real-time video stream. The third region 30 is the so-called imaging modality room in which the subject S is radiologically imaged. If the imaging device acquires the image by scanning, the subject S is scanned in the third region 30. The third acquisition device 18 is another 360° camera located at the third region 30, in particular in the imaging modality room.

The first to third acquisition devices 14, 16, 18 may be stereoscopic cameras. Moreover, the acquisition system, in particular, the first acquisition device 14, is configured to transmit the first video stream 20, for example by wired or wireless communication means such as a (wireless) local area network, to the controller 12 in real-time. Analogously, the second acquisition device 16 and the third acquisition device 18 are configured to transmit the second video stream 20 and the third video stream 22, respectively, to the controller 12 in real-time.

The controller 12 may be provided with a streaming server unit configured for providing the receiving and transmission functionality of the controller 12, for example. In particular, the controller 12 is configured to receive the first, second and third video streams 20, 22, 24 from the acquisition system. Upon receiving, by the controller 12, a video selection signal from the user interface system 50 based on a first user command which indicates that the user 70 selects one of the video streams, for example the second video stream 22, for output on the immersive display 54, the controller 12 selects the at least one first video, and transmits only this video stream 22 to the user interface system 50, which video stream 22 is thus, in response to the first user command, replayed on the immersive display 54 to the user 70 in real-time. Accordingly, the user is provided with video and sound from the examination room 28 in real-time, so that she/he can quickly and efficiently assess any deviation from a desired standard operating procedure. Similar effects can be achieved when the user selects another one of the video streams or even a plurality of video streams.

The immersive display 54 can display any of monoscopic images/videos and stereoscopic images/videos. In the herein described embodiment, the image acquisition devices 14, 16, 18 each acquire a monoscopic image/video, and the immersive display 54 is formed as virtual reality goggles. Thus, the virtual reality goggles may comprise a video processor and positional and rotational sensors configured to detect a movement of the goggles and to determine which section of the acquired region of the diagnostic site D is currently located within the field of view of the virtual reality goggles. Accordingly, display portions of the virtual reality goggles are configured to display the video data of the video stream 22 corresponding to this section. Further, when the virtual reality goggles are moved so that the position/posture of the goggles changes, the section of the acquired region changes accordingly, so that the display portions will, based on control by the video processor and the positional and rotational sensors, correspondingly display the video data of the video stream 22 corresponding to the changed section.

The user thus receives a more complete and accurate overview of the ongoing examination procedure, and can interact with the medical staff on site through the at least one user communication input unit 52. Therefore, the at least one user communication input unit 52 is configured to receive a user input with audio input and a video input and to generate a user input signal representative of the user input. This user input is communicated to at least one of the user communication output units 62, 64, 66, for example, the user communication output unit 64 located at the region at which the first acquisition device 16 is located the medical technician T in the examination room 28 receives the user input. Specifically, the user interface system 50 transmits the user input signal to the controller 12, and the controller 12 receives the user input signal and transmits it to the user communication output unit 64 simultaneously with transmitting the at least one video stream to the user interface system 50 for output of the user input at the diagnostic site D.

In addition to the Radiological Information System 72, RIS, the Picture Archiving and Communication System 74, PACS, and the a medical imaging device 76 (which can exemplarily be understood to be an X-ray CT imaging device), the system 10 comprises as further systems a patient positioning assistance system (not shown) and an intravascular contrast agent injector 78. The patient positioning assistance system may comprise a subject position detecting unit configured for detecting the position of the subject S, and a user assistance output unit for outputting a signal with instructions on how to adapt the subject's S position to a desired position. The intravascular contrast agent injector 78 comprises a display screen 88 configured for displaying injection parameters. All of these radiological systems are configured to transmit respective radiological system video feeds 92, 94, 96, 98 of their display screens 82, 84, 86, 88 to the controller 12.

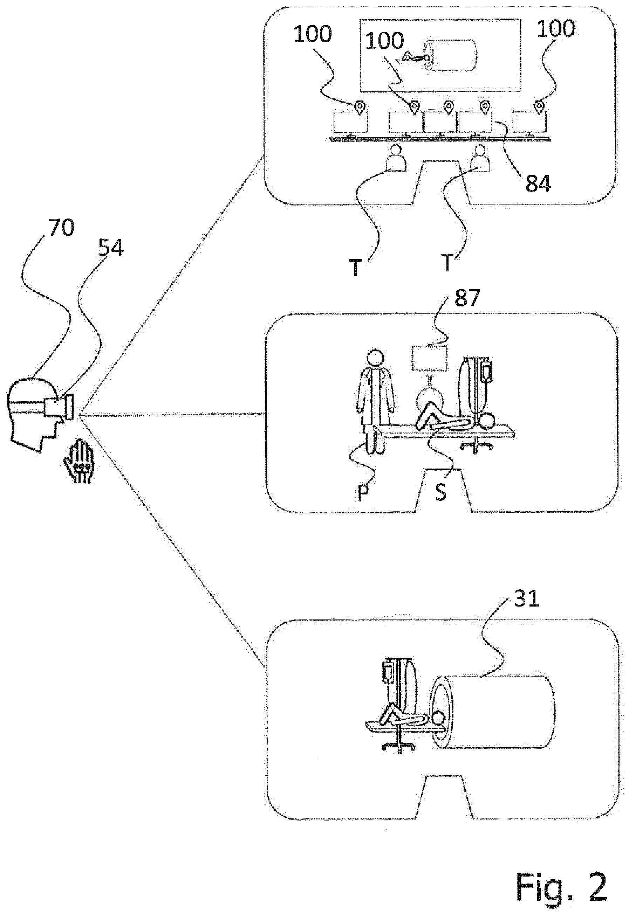

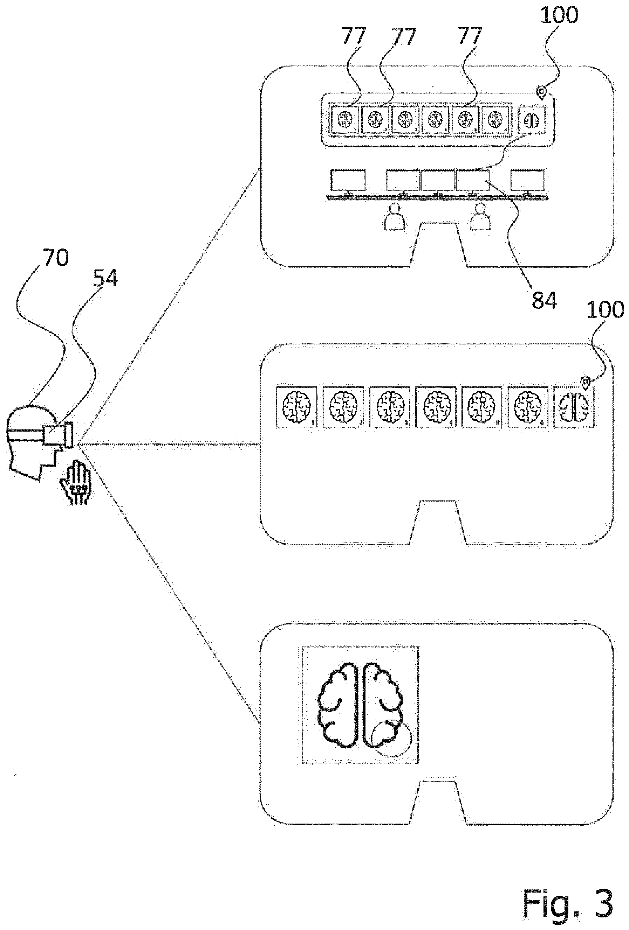

The at least one user input communication unit 52 comprises a remote controlling device for the user 70, by which the user 70 may input a second user command for selecting one of the aforementioned radiological systems, for example, the PACS 74. Upon receiving this second user command, the at least one user input communication unit 52 transmits a radiological system selection signal based on the selection, which signal is, in this embodiment, indicative for the selection of the PACS. In response to receiving the radiological system selection signal by the controller 12, the controller performs processing of the video stream 22 by overlaying the radiological system video feed 94 corresponding to the PACS onto the video stream 22 in response to receiving the radiological system selection signal to augment the video stream 22. The augmented video stream 22 is then conveyed to the immersive display 54 where it is displayed to be viewed by the user 70 (cf. Fig. 3). Alternatively, if the video stream 20 of the preparation area 26 is displayed on the immersive display 54, the controller 12 can overlay a video feed from the patient positioning assistance system displaying a graphical representation indicating how the position of the patient should be changed. For example, the first video feed 20 can be augmented by displaying a graphical field 87 with the text "elevate knee" (cf. Fig. 2).

The remote controlling device can be formed as one or more virtual reality gloves or a pointer communicating with the immersive display 54 (virtual reality goggles), so that a cursor can be displayed on the display portions. To input the first user command, the user 70 may thus select a graphical representation displayed on the immersive display 54 corresponding to the examination room 28, whereas to input the second user command, the user may select, such as by means of the cursor, a visualized (e.g., by an icon) or invisible area in the displayed video associated with the respective radiological system (herein, the PACS 74). Such visualized area is exemplarily illustrated in Fig. 2 showing the video stream of the examination room at the top. At the top right corner of each of the displays present in the region 28 of the diagnostic site D, an icon 100 is shown. By selecting the icon 100 of the display 84 associated with the desired radiological system, the PACS 74, by means of the remote controlling device, the user 70 can trigger the overlay of the radiological system video feed 94 onto the video stream 22. The augmented video stream 22 is then displayed to the user 70 so that acquired images 77 obtained from the PACS are displayed arranged side by side (e.g., in an assumed horizontal cross-section, along a circular arc), as exemplarily shown in Fig. 3. If the remote controlling device comprises the virtual reality glove/s, the user 70 may further be provided with haptic feedback when inputting the first and/or second user commands. Also, the virtual reality gloves may provide tactile feedback and enable a higher degree of intuitiveness.

In addition, in the augmented video stream 22, a further icon 100 is overlaid over the video stream 22. By selecting this icon 100 by the remote controlling device, the user 70 can trigger the controller 12 to refrain from transmitting any of the video streams 20, 22, 24, but instead transmit only the radiological system video feed 94 to the user interface system 50 to be displayed on the immersive display 54. A further icon 100 can be overlaid by the controller 12 onto the radiological system video feed 94, the selection of which icon 100 overlaid onto the radiological system video feed 94 triggers the controller 12 to zoom in on a section of the radiological system video feed 94 at which the icon 100 is arranged (see Fig. 3, bottom right).

Furthermore, the controller 12 is optionally configured to process the video streams 20, 22, 24 for anonymization of the subject S (patient), the medical technicians T and a physician P present in the preparation room 26 appearing in the video stream before transmitting the at least one video stream to the user interface system 50. In the present embodiment, these persons are shown on the immersive display 54 anonymized as avatars. In addition, to provide improved privacy, the controller 12 may be configured for limiting access of the user interface system 54 to predefined video stream/s under consideration of the user 70 logged in to the user interface system 50.

As has become apparent from the above, the telepresence system according to the present disclosure enables better communication, collaboration and interaction of multiple stakeholders in the radiological workflow, while eliminating location dependency, and may additionally provide one or more of the following advantages:
The remote physician can immersively monitor the preparation of the patient and can see in real-time the optimal instructions for positioning, and hence advise the preparer through the created virtual environment in case the position of the patient needs to be adjusted. Furthermore, the remote user can communicate with the patient in order to calm her/him down and prepare her/him for the examination procedure. Vice versa, the medical technician on-site can immersively communicate with the remote user, understand and interpret the SOP defined by the user without requiring the user to be physically present at the diagnostic center. The user can also monitor the image quality of the scan and react to spontaneous needs for adjustments in the examination procedure while the patient is still in the diagnostic center. Through the immersive scene and the live data of the examination results, the user can ask the technician to reacquire certain images or perform certain procedures again if not satisfied with their quality or if a need for further images arises during the examination of the results. All of the above reduces the risk of having to re-order the patient and repeat the examination process. The user also can be virtually present and give real time support in emergency cases by switching between different immersive scenes of the diagnostic site/center. The real-time status of the radiological interfaces and devices provided by the video stream may enable the user to gain more complete knowledge of the patient examination process and intervene, if necessary.

Moreover, the user or specialist can better understand the progress of the examination process by being immersed in the provided virtual diagnostic environment through the visual and optionally audio feedback. The user can communicate and support the medical staff and take control of PACS, RIS and other medical interfaces in real-time which can also be further improved through tactile feedback without being location-dependent. The video stream of the examination room and control at the modality interface may allow the remote user to compare the current acquired images with previous results of the same patient in real-time without waiting for the current results to be available in PACS. Through the audio and visual communication enabled by the telepresence system, the user, if not present at the diagnostic center, can interact with the patient, help the patient in preparation and calm him down during the examination process.

With the provided telepresence, remote technical support can not only control the displays but also virtually monitor and take control of any aspect of the examination environment regardless of their location. Technical support can either immersively communicate a required solution to the technician on-site or take control of the various interfaces to solve an arising problem. Moreover, the user can not only access the acquired images but also be virtually present in high demand areas, and provide assistance to multiple examinations in different hospitals such as define the correct protocols to be applied or prioritize the patients that need urgent care. Further, the interactive virtual environment may enable a trainee and her/his trainer to remotely participate in the examinations, perform hospitations and recurrent training in different centers without the limitation of travelling to the diagnostic center itself. The trainer, if virtually present, can support the trainee and intervene when needed without having to be physically present in the center. Also, the trainee can virtually and immersively participate in special or rare examinations in different centers around the world as a part of the development of new skills.

It is believed that the advantages of the technique presented herein will be fully understood from the foregoing description, and it will be apparent that various changes may be made in the form, constructions and arrangement of the exemplary aspects thereof without departing from the scope of the disclosure or without sacrificing all of its advantageous effects. Because the technique presented herein can be varied in many ways, it will be recognized that the disclosure should be limited only by the scope of the claims that follow.

## Claims

1. A telepresence system (10) for teleradiology, comprising:
an acquisition system located at a radiological diagnostic site and comprising one or more acquisition devices (14, 16, 18), wherein each acquisition device (14, 16, 18) is configured to acquire a respective video stream (20, 22, 24) comprising video data acquired of a region (26, 28, 30) of the diagnostic site at which the respective acquisition device (14, 16, 18) is located, and wherein the acquisition system is configured to transmit the one or more video streams (20, 22, 24) to a controller (12);
a user interface system (50) located remotely from the diagnostic site, wherein the user interface system (50) comprises a user communication input unit (52) and a replay unit with an immersive display (54); and
a user communication output system (62, 64, 66) located at the diagnostic site,
wherein the controller (12) is configured to receive the one or more video streams (20, 22, 24) and to transmit at least one of the video streams to the user interface system (50), wherein the replay unit is configured to output the at least one video stream on the immersive display (54) to a user (70), and
wherein the user communication input unit (52) is configured to receive a user input and generate a user input signal representative of the user input, wherein the user interface system (50) is configured to transmit the user input signal to the controller (12), and the controller (12) is configured to receive the user input signal and to transmit the user input signal to the user communication output system (62, 64, 66) for output of the user input at the diagnostic site.

2. The system of claim 1,
wherein the user input comprises one or more of the following: a textual input, an audio input and a video input; and/or
wherein the one or more video streams (20, 22, 24) further comprise audio data acquired of the region of the diagnostic site at which the respective acquisition device (14, 16, 18) is located.

3. The system of any one of claims 1 and 2, wherein the acquisition devices (14, 16, 18) are configured to acquire the video streams (20, 22, 24) as real-time video streams and to transmit the video streams (20, 22, 24) via the controller (12) to the replay unit in real-time, and wherein the replay unit is configured to output the at least one video stream in real-time.

4. The system of any one of claims 1 to 3, wherein the controller (12) is configured to transmit the at least one video stream to the user interface system (50) simultaneously with transmitting the user input signal to the user communication output system (62, 64, 66).

5. The system of any one of the preceding claims, wherein at least one of the acquisition devices (14, 16, 18) is configured for acquiring
video data of at least a half sphere, preferably a full sphere, around the acquisition device (14, 16, 18), and/or
stereoscopic image data so that the video data is stereoscopic video data.

6. The system of any one of the preceding claims,
wherein the immersive display (54) comprises virtual reality goggles; and
wherein the immersive display (54) is preferably a stereoscopic display.

7. The system of any one of the preceding claims,
wherein the acquisition devices (14, 16, 18) are arranged at the diagnostic site so as to acquire video data, and optionally audio data, of different areas of the diagnostic site,
wherein the different areas are preferably different rooms of the diagnostic site.

8. The system of claim 7, wherein the different areas comprise a preparation area adapted for preparing a subject for radiological examination, an examination room adapted for controlling the examination, and an imaging modality room adapted for radiologically imaging the subject.

9. The system of any one of the preceding claims, further comprising one or more radiological systems (72, 74, 76, 78) located at the radiological diagnostic site,
wherein the one or more radiological systems are each configured to transmit a radiological system video feed of a display screen (82, 84, 86, 88) of the respective at least one radiological system (72, 74, 76, 78) to the controller (12), and
wherein the controller (12) is configured to overlay the radiological system video feed on the at least one video stream to be transmitted to the user interface system (50) so as to augment the at least one video stream.

10. The system of claim 9,
wherein the one or more radiological systems (72, 74, 76, 78) comprise one or more of the following:
a medical imaging device (76), in particular an X-ray CT imaging device, an MRT imaging device, a PET-CT imaging device, a PET-MRT imaging device, or an ultrasonic imaging device;
a Radiological Information System, RIS (72);
a Picture Archiving and Communication System (74), PACS;
a patient positioning assistance system, preferably comprising a subject position detecting unit configured for detecting the position of the subject (S), and a user assistance output unit for outputting a signal with instructions on how to adapt the subject's (S) position to a desired position; and
an intravascular injector (78), in particular, a contrast agent injector.

11. The system of claim 9 or 10, wherein one of the radiological systems is a Picture Archiving and Communication System (74), PACS, and wherein the radiological system video feed is overlayed on the at least one video stream such that radiological images (77) are arranged side by side.

12. The system of any one of the preceding claims,
wherein the user interface system (50) is configured to receive a first user command for selecting the at least one video stream from among the video streams, and to transmit, to the controller (12), a video selection signal based on the first user command, and
wherein the controller (12) is configured to select the at least one first video based on the video selection signal.

13. The system of any one of claims 9 to 12,
wherein the user interface system (50) is configured to receive a second user command for selecting one of the one or more radiological systems and to transmit, to the controller (12), a radiological system selection signal based on the selection, and
wherein the controller (12) is configured to overlay the radiological system video feed on the at least one video stream in response to receiving the radiological system selection signal.

14. The system of any one of the preceding claims, wherein the controller (12) is further configured to process the at least one video stream for anonymization of persons appearing in the video stream before outputting the processed at least one video stream on the immersive display (54).

15. Telepresence method for teleradiology, comprising the steps of:
acquiring, by each of one or more acquisition devices (14, 16, 18) of an acquisition system located at a radiological diagnostic site, a respective video stream, wherein each video stream comprises video data acquired of a region of the diagnostic site at which the respective acquisition device (14, 16, 18) is located;
transmitting, by the acquisition system, the one or more video streams to a controller (12);
receiving, by the controller (12), the one or more video streams;
transmitting, by the controller (12), at least one of the video streams to a user interface system (50) located remotely from the diagnostic site;
outputting, by a replay unit of the user interface system (50), the at least one video stream on an immersive display (54) of the replay unit to a user (70);
receiving, by a user communication input unit (52) of the user interface system (50), a user input;
generating, by the user communication input unit (52), a user input signal representative of the user input;
transmitting, by the user interface system (50), the user input signal to the controller (12);
receiving, by the controller (12), the user input signal; and
transmitting, by the controller (12), the user input signal to a user communication output system (62, 64, 66) located at the diagnostic site for output at the diagnostic site.
